(19) Europäisches Patentamt / European Patent Office / Office européen des brevets

(11) **EP 3 251 595 A1**

(12) **EUROPÄISCHE PATENTANMELDUNG**

(43) Veröffentlichungstag:
**06.12.2017 Patentblatt 2017/49**

(51) Int Cl.:
*A61B 5/05* (2006.01)  *A61B 5/145* (2006.01)
*A61K 49/18* (2006.01)  *B82Y 5/00* (2011.01)

(21) Anmeldenummer: **17173392.6**

(22) Anmeldetag: **30.05.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(30) Priorität: **30.05.2016 DE 102016109926**

(71) Anmelder: **BIOTRONIK SE & Co. KG**
**12359 Berlin (DE)**

(72) Erfinder:
• **Ulmer, Jens**
**8700 Küsnacht (CH)**
• **Lummer, Ingo**
**8400 Winterthur (CH)**

(74) Vertreter: **Keck, Hans-Georg**
**BIOTRONIK SE & Co. KG**
**Corporate Intellectual Properties**
**Woermannkehre 1**
**12359 Berlin (DE)**

(54) **SENSORSYSTEM, INSBESONDERE ZUM BESTIMMEN EINER GLUCOSEKONZENTRATION**

(57) Ein medizinisches Sensorsystem (1) zum Bestimmen eines Merkmals in einem tierischen oder menschlichen Körper, z.B. der Glucosekonzentration, weist eine Mehrzahl an magnetischen Nanopartikeln (10) auf, die jeweils dazu ausgebildet sind, durch Eingehen von reversiblen chemischen Bindungen mit einem weiteren Stoff ihr magnetischen Relaxationsverhaltens zu ändern. Weiterhin weist das Sensorsystem (1) eine Mehrzahl an magnetischen Referenz-Nanopartikeln (20) auf, die die besagte chemische Bindung nicht eingehen oder in einem geringeren Ausmaß als die Nanopartikel (10) eingehen.

Fig. 2

EP 3 251 595 A1

**Beschreibung**

Die vorliegende Erfindung betrifft ein Sensorsystem zur Bestimmung eines Merkmals, insbesondere der Konzentration eines Analyten, insbesondere Glucose, in einem tierischen oder menschlichen Körper sowie ein entsprechendes Verfahren.

In der Medizin kommen Sensorsysteme zum Einsatz, bei denen zumindest Teile des Systems direkt in einen Körper eines Patienten eingesetzt bzw. implantiert werden, um möglichst genau und unmittelbar physiologische Ist-Zustände, insbesondere Konzentrationen von bestimmten Analyten, zu erfassen.

Aus der EP 2 433 562 B1 ist ein medizinisches Sensorsystem zum Nachweis von zumindest einem Merkmal in einem tierischen oder menschlichen Körper bekannt, das eine in den Körper implantierbare Signalaufnahmeeinheit und eine räumlich von der Signalaufnahmeeinheit getrennt angeordnete Signalverarbeitungseinheit mit einem Sender und einem Empfänger aufweist, wobei der Sender dazu ausgebildet ist, ein magnetisches Wechselfeld zum Einwirken auf die Signalaufnahmeeinheit auszusenden und der Empfänger dazu ausgebildet ist, ein Antwortsignal der Signalaufnahmeeinheit zu empfangen, das durch die magnetische Wechselwirkung des magnetischen Wechselfelds mit der Signalaufnahmeeinheit generierbar ist. Insbesondere ist vorgesehen, dass das Antwortsignal von einem Relaxationssignal zumindest eines Nanopartikels gebildet wird und durch eine Änderung eines Bindungsverhaltens zwischen dem zumindest einen Nanopartikel und einem weiteren Bestandteil des Sensorsystems verursacht wird, wobei das Bindungsverhalten abhängig von einer Konzentration eines Analyten ist, die es zu bestimmen gilt.

Hierbei kann es jedoch unter Umständen zu einer fehlerhaften Bestimmung der Analytkonzentration kommen, da sich äußere, nicht analytbezogene Einflüsse bemerkbar machen können. So ist z. B. das in der EP 2 433 562 B1 beschriebene System anfällig hinsichtlich der lokalen Partikelkonzentration am Messort, der relativen Position (insbesondere Abstand) der Nanopartikeln und des Messaufnehmers, der Viskosität am Messort und der Temperatur. Diese Parameter ändern sich in der Regel fortwährend und verursachen eine Drift der Sensorkennlinie, was eine Rekalibrierung des Sensors erforderlich macht.

Hiervon ausgehend liegt der vorliegenden Erfindung die Aufgabe zugrunde ein Sensorsystem bzw. ein Verfahren der eingangs genannten Art im Hinblick auf die vorgenannte Problematik zu verbessern.

Diese Aufgabe wird durch ein Sensorsystem mit den Merkmalen des Anspruchs 1 sowie durch ein Verfahren mit den Merkmalen des Anspruchs 12 gelöst.

Vorteilhafte Ausgestaltungen der beiden Aspekte der Erfindung sind in den entsprechenden Unteransprüchen angegeben und werden nachfolgend beschrieben.

Gemäß Anspruch 1 wird ein medizinisches Sensorsystem zur Bestimmung eines Merkmals, insbesondere der Konzentration eines Analyten, in einem tierischen oder menschlichen Körper, vorgeschlagen, mit einer Mehrzahl an magnetischen Nanopartikeln, die jeweils dazu ausgebildet sind, reversible chemische Bindungen mit einem weiteren Stoff einzugehen, wobei diese Bindungen zu einer Veränderung des magnetischen Relaxationsverhaltens des jeweiligen Nanopartikels in einem magnetischen Wechselfeld fuhren.

Erfindungsgemäß ist nun vorgesehen, dass das Sensorsystem weiterhin eine Mehrzahl an magnetischen Referenz-Nanopartikeln aufweist, die die besagte chemische Bindung nicht eingehen oder in einem geringeren Ausmaß eingehen, so dass deren magnetisches Relaxationsverhalten im Idealfall unabhängig von dem besagten Merkmal bzw. der Konzentration des Analyten ist oder zumindest in einem geringeren Maße von diesem Merkmal bzw. der Konzentration des Analyten abhängt, als das magnetische Relaxationsverhalten der magnetischen Nanopartikel, so dass insbesondere durch magnetische Suszeptometrie gemessene Werte der Referenz-Nanopartikel zum Herausrechnen von Störeinflüssen verwendbar sind, z.B. hervorgerufen durch Temperaturdrift (siehe oben).

Der Umstand, dass die Referenz-Nanopartikeln die besagte chemische Bindung in einem geringeren Ausmaß eingehen, bedeutet mit anderen Worten, dass die Referenz-Nanopartikel eine geringere Bindungsaffinität (z.B. beschreibbar durch die Dissoziationskonstante $K_D$) hinsichtlich des besagten weiteren Stoffes aufweisen, wobei bevorzugt diese Bindungsaffinität der Referenz-Nanopartikel weniger als 10% der Bindungsaffinität der Nanopartikel hinsichtlich des besagten weiteren Stoffes beträgt.

Die Referenz-Nanopartikel bilden also ein Referenzsystem, das in ähnlicher Weise wie die Nanopartikel den Umgebungseinflüssen, Messbedingungen etc. ausgesetzt ist, jedoch nicht oder nur in geringem Maße am Nachweis des Merkmals teilnimmt. Auf diese Weise können z. B. durch Verhältnisbildung von gemessenen Werten (z.B. Relaxationssignale) Störeinflüsse mit Vorteil herausgerechnet werden.

Im Rahmen der vorliegenden Erfindung soll unter einem "magnetischen Nanopartikel" bzw. "magnetischen Referenz-Nanopartikel" insbesondere ein Partikel bzw. Teilchen verstanden werden, das eine Erstreckung und/oder einen Durchmesser im Bereich von 1 nm bis 500nm aufweist, wobei der Durchmesser des jeweiligen Nanopartikels bevorzugt größer ist als 11,2nm (siehe unten).

Zudem weist das Nanopartikel bzw. Referenz-Nanopartikel einen magnetischen Stoff, wie beispielsweise Verbindungen aus Eisen, Nickel, Kobalt, Chrom und/oder Legierungen aus Mangan mit Aluminium, Kupfer, Zinn, Antimon, Arsen, Wismut, Bor oder jeden anderen Stoff, jede andere Verbindung, jede andere Legierung, die der Fachmann für sinnvoll hält, auf.

Vorteilhafterweise weist das Sensorsystem viele Nanopartikel/Referenz-Nanopartikel auf und/oder vorteilhaft jeweils eine Massenkonzentration bzw. eine Masse pro Volumen Sensoreinheit bzw. Signalaufnahmeeinheit (siehe unten) zwischen 0,01-100 mg/ml, bevorzugt zwischen 0,1-50 mg/ml und besonders vorteilhaft zwischen 1-10 mg/ml auf. Eine Massenkonzentration von 1 mg/ml entspricht beispielsweise bei einem angenommenen Teilchendurchmesser von 20 nm einer Teilchenzahl von $3,3x10^{15}$.

In einer weiteren vorteilhaften Ausgestaltung der Erfindung wird vorgeschlagen, dass das magnetische Nanopartikel/Referenz-Nanopartikel $Fe_3O_4$ umfasst bzw. aus monodispersem Eisenoxid besteht, wodurch es besonders vielseitig einsetzbar und biokompatibel ist.

Gemäß einer besonders bevorzugten Ausführungsform ist vorgesehen, dass das besagte, zu bestimmende Merkmal die Konzentration eines Analyten ist, insbesondere Glucose, wobei die magnetischen Nanopartikel jeweils dazu ausgebildet sind, in Gegenwart des Analyten in Abhängigkeit von der Konzentration des Analyten jene reversiblen chemischen Bindungen mit einem weiteren Stoff einzugehen, wobei diese Bindungen zu der Veränderung des magnetischen Relaxationsverhaltens des jeweiligen Nanopartikels führen.

In diesem Zusammenhang soll insbesondere unter einem "Analyt" das Molekül bzw. der Stoff verstanden werden, das bzw. der als Merkmal von dem Sensorsystem im tierischen und/oder menschlichen Körper nachgewiesen werden soll bzw. dessen Konzentration bestimmt werden soll.

Abgesehen von der Glucosekonzentration, kann unter einem "Merkmal" insbesondere ein Parameter, wie ein pH-Wert, eine Ladung, beispielsweise eines Ions, eine Temperatur, eine Größe, eine Masse, ein Aggregatszustand, eine An-. bzw. Abwesenheit und/oder insbesondere eine Menge eines Stoffs, wie ein Salz oder ein Mineralstoff (z.B. Kalzium, Phosphat, Kalium, Schwefel, Natrium, Chlorid, Magnesium, Eisen, Iodid, Mangan, Kupfer, Kobalt, Zink, Fluorid, Selen und/oder Chrom), ein Ion (z.B. Wasserstoff, Natrium, Kalium, Ammonium, Hydronium, Magnesium, Kalzium, Eisen II, Eisen III, Fluorid, Chlorid, Jod, Hydroxyl, Nitrat, Bicarbonat, Oxid, Sulfat und/ oder Phosphat), ein Zucker oder eine Zuckerverbindung (z.B. Glucose, Saccharose, Fructose, Lactose, Maltose, Dextrose und/oder Glykogen) oder ein Protein, und/oder eines Analyten verstanden werden. Generell wäre jedoch auch jedes andere, dem Fachmann als zweckdienlich erscheinende Merkmal denkbar solange dessen Gegenwart das Relaxationsverhalten der Nanopartikel beeinflusst. Bevorzugt bezieht sich das Merkmal auf einen variablen Bestandteil des tierischen und/oder menschlichen Körpers.

Gemäß einer bevorzugten Ausführungsform des erfindungsgemäßen Sensorsystems ist vorgesehen, dass der besagte weitere Stoff einen Rezeptor für den Analyten bildet.

Unter einem Rezeptor wird im Rahmen der Erfindung insbesondere ein Molekül und/oder einen Stoff verstanden, das bzw. der den Analyten und durch die Ähnlichkeit des Analogons (siehe unten) zum Analyten auch das Analogon spezifisch bindet. Dabei kann der Rezeptor aus der Stoffklasse der Peptide, Proteine- insbesondere der Antikörper und deren Fragmente, RNA, DNA - insbesondere Aptamere und deren Verwandte, zyklische Makromoleküle - wie insbesondere Moleküle, die selektiv Ionen binden bzw. Ionophore, nichtzyklische Makromoleküle - d. h. Polymere, deren Erkennungsmerkmal während einer Polymerisation eingeprägt wurde bzw. sogenannte "molecular imprinted polymers" - und/oder aus jeder anderen, dem Fachmann für sinnvoll erscheinende Stoffklasse stammen.

Weiterhin soll unter dem Begriff "reversibel" hier insbesondere umkehrbar bzw. (auf) lösbar verstanden werden.

Weiterhin ist gemäß einer bevorzugten Ausführungsform des erfindungsgemäßen Sensorsystems vorgesehen, dass die magnetischen Nanopartikel jeweils einen magnetischen Kern aufweisen, wobei die Kerne jeweils mit einem Analogon des Analyten beschichtet sind, so dass sich die magnetischen Nanopartikel mit dem jeweiligen Analogon in Abhängigkeit von der Konzentration des Analyten an den Rezeptor des Analyten und des Analogons reversibel chemisch binden. Gemäß einer bevorzugten Ausführungsform sind die magnetischen Nanopartikel mit dem jeweiligen Analogon in Abhängigkeit von der Konzentration des Analyten an den Rezeptor des Analyten und des Analogons reversibel durch Physisorption gebunden.

Hierbei soll weiterhin unter einem "Analogon" insbesondere ein Molekül und/oder ein Stoff verstanden werden, das bzw. der aufgrund seiner Strukturähnlichkeit, ähnlicher Ladungsverteilung und/oder eines anderen, dem Fachmann für zweckmäßig erscheinenden Kriteriums von dem gleichen Rezeptor gebunden wird.

Weiterhin ist gemäß einer bevorzugten Ausführungsform des erfindungsgemäßen Sensorsystems vorgesehen, dass das Analogon ein Dextran ist und/oder dass der Rezeptor Concanavalin-A ist. Vorzugsweise weist das Dextran ein Molekulargewicht zwischen 40.000 Da und 200.000 Da auf.

Es ist natürlich gemäß einer Ausführungsform auch möglich, dass die Kerne der Nanopartikel mit dem Rezeptor des Analyten beschichtet sind. Das Sensorsystem bzw. die Signaiaufhahmeeinheit weist dann weiterhin das Analogon auf. Hier findet also eine Verdrängungsreaktion statt, bei der der Analyt das Analogon vom Rezeptor verdrängt, wenn es in entsprechender Konzentration vorhanden ist.

Entsprechend ist gemäß einer Ausführungsform vorgesehen, dass die Signalaufnahmeeinheit (siehe unten) im Fall des mit Analogon beschichteten Nanopartikels zusätzlich den Rezeptor aufweist und im Falle des mit Rezeptor beschichteten Nanopartikels das Analogon. Hierbei sind diese bevorzugt an einer festen Struktur, wie beispielsweise einer Netz- oder Gitterstruktur, angeordnet. Durch die Anwesenheit dieser Gegenspieler kommt es zu einer reversiblen Bindung zwischen dem Analogon und dem Rezeptor.

Der Nachweis des Analyten beruht nun auf einer Verdrängungsreaktion (Kerne sind mit dem Rezeptor beschichtet), bei dem der Analyt das Analogon vom Rezeptor verdrängt bzw. auf einer kompetitiven Bindung des Analyten an den Rezeptor (Kerne sind mit dem Analogon beschichtet). Ändert sich im untersuchten Gewebe die lokale Konzentration des Analyten, wird die Anzahl der Bindungen zwischen dem Analogon und dem Rezeptor reduziert und eine Bewegungs- oder Rotationsfreiheit und/oder insbesondere eine magnetische Relaxation des funktionalisierten magnetischen Nanopartikels verändert bzw. erhöht. Diese kann in einem magnetischen Wechselfeld mit einer magnetischen Flussdichte mit beispielsweise einer Amplitude von 0,5 mT gemessen werden. Unter einem "magnetischen Wechselfeld" wird im Rahmen der vorliegenden Erfindung insbesondere ein magnetisches Feld verstanden, das in Stärke und/oder Polung wechselt und das z.B. durch eine Wechselspannung bzw. -strom hervorgerufen wird.

Weiterhin ist gemäß einer bevorzugten Ausführungsform des erfindungsgemäßen Sensorsystems vorgesehen, dass der Analyt Glucose ist, d. h., die Signalaufnahmeeinheit (siehe unten) ist als Sensor für den Analyten Glucose ausgebildet.

Weiterhin ist gemäß einer bevorzugten Ausführungsform des erfindungsgemäßen Sensorsystems vorgesehen, dass die magnetischen Referenz-Nanopartikel jeweils ebenfalls einen magnetischen Kern aufweisen, der jeweils mit PEG, insbesondere PEG300, beschichtet ist.

Im Gegensatz zu den Nanopartikeln sind die Referenz-Nanopartikel nicht mit dem Analogon/Rezeptor beschichtet bzw. solchermaßen funktionalisiert, damit sie an der Nachweisreaktion idealerweise nicht teilnehmen. Ihr Relaxationsverhalten ändert sich entsprechend nur gemäß der allgemeinen Umgebungsbedingungen, so dass sie das besagte Referenzsystem bilden können.

Weiterhin ist gemäß einer bevorzugten Ausführungsform des erfindungsgemäßen Sensorsystems vorgesehen, dass das Sensorsystem des Weiteren eine in einen tierischen oder menschlichen Körper implantierbare Signalaufnahmeeinheit aufweist, die die Nanopartikel, Referenz-Nanopartikel und den besagten Stoff bzw. Rezeptor aufweist, sowie vorzugsweise eine räumlich von der Signalaufnahmeeinheit getrennt anordenbare, insbesondere ex vivo anordenbare Signalverarbeitungseinheit mit einem Sender und einem Empfänger, wobei der Sender dazu ausgebildet ist, ein magnetisches Wechselfeld zum Einwirken auf die Nanopartikel und Referenz-Nanopartikel der Signalaufnahmeeinheit auszusenden, und wobei der Empfänger dazu ausgebildet ist, ein Antwortsignal in Form eines Relaxationssignals der Signalaufnahmeeinheit zu empfangen, das durch die magnetische Wechselwirkung des magnetischen Wechselfelds mit den magnetischen Nanopartikeln und den magnetischen Referenz-Nanopartikeln der Signalaufnahmeeinheit generierbar ist.

Weiterhin ist gemäß einer bevorzugten Ausführungsform des erfindungsgemäßen Sensorsystems vorgesehen, dass das Sensorsystem eine mit der Signalverarbeitungseinheit verbundene Recheneinheit aufweist, die dazu ausgebildet ist, zur Berechnung der Konzentration des Analyten zumindest den Imaginärteil der dynamischen Suszeptibilität der magnetischen Nanopartikel und der Referenz-Nanopartikel mit Hilfe des Antwortsignals für eine Vielzahl an Frequenzen des magnetischen Wechselfeldes zu berechnen, wobei die Recheneinheit dazu konfiguriert ist, zur Berechnung der Konzentration des Analyten ein Verhältnis zwischen der Amplitude eines den Referenz-Nanopartikeln zugeordneten ersten Peaks des Imaginärteils der dynamischen Suszeptibilität und der Amplitude eines den Nanopartikeln zugeordneten zweiten Peaks des Imaginärteils der dynamischen Suszeptibilität zu bestimmen. Hierbei bezieht sich der "Imaginärteil der dynamischen Suszeptibilität" insbesondere auf den mathematischen Betrag eines solchen Imaginärteils.

Vorzugsweise ist gemäß einer Ausführungsform der Erfindung vorgesehen, dass die Partikelgröße bzw. der Durchmesser der Nanopartikel bzw. Referenz-Nanopartikel so gewählt wird, dass die Neel Relaxation vernachlässigbar ist. Dies ist der Fall, wenn die Zeitskala der Messungen kürzer ist als die Neel Relaxationszeiten. Besonders bevorzugt werden daher Nanopartikel bzw. Referenz-Nanopartikel verwendet, deren Durchmesser größer ist als 11,2 nm.

Weiterhin ist gemäß einer bevorzugten Ausführungsform des erfindungsgemäßen Sensorsystems vorgesehen, dass die Signalaufnahmeeinheit zumindest ein Hydrogel aufweist, das insbesondere von einem Copolymer gebildet ist, wobei die Nanopartikel, die Referenznanopartikel und der besagte Stoff bzw. Rezeptor in dem Hydrogel angeordnet sind. Hierbei kann der weitere Stoff bzw. der Rezeptor an das Hydrogel gebunden sein. Für den Fall, dass die Nanopartikel den Rezeptor als Beschichtung aufweisen, ist bevorzugt das Analogon im Hydrogel vorgesehen bzw. daran gebunden.

Bei einem Hydrogel handelt es sich um ein Gel aus einem Polymer bzw. Copolymer, das Wasser enthält aber wasserunlöslich ist. Ferner sind insbesondere dessen Moleküle chemisch, z.B. durch kovalente oder ionische Bindungen, oder physikalisch, z. B. durch Verschlaufen von Polymerketten, zu einem dreidimensionalen Netzwerk verknüpft.

Das Hydrogel hat bevorzugt eine Konsistenz, die ein einfaches subkutanes Injizieren des Hydrogels erlaubt. Hierbei wird das Hydrogel bevorzugt als Dispersion von 200 bis 300 Mikrometer großen Gelkügelchen suspendiert in einer zu Injektionszwecken geeigneten Lösung unter der Haut platziert.

Grundsätzlich wäre es jedoch auch möglich, dass das Hydrogel erst in situ beispielsweise durch einen Temperaturwechsel geliert. Durch das Hydrogel kann die Signalaufnahmeeinheit bzw. der Biosensor besonders einfach und anwenderfreundlich mittels Injektion ins Gewebe platziert werden. Zudem treten patientenfreundlich und gesundheitsfördernd sehr geringe bis keine Fremdkörperreaktion nach der Implantation auf. Des Weiteren bleiben durch die reduzierte bis ausbleibende Fremdkörperreaktion bzw. Entzündungsreaktion Bedingungen an der Implantationsstelle unbeeinflusst, wodurch optimale Nachweisbedingungen herrschen.

Weiterhin ist gemäß einer bevorzugten Ausführungsform des erfindungsgemäßen Sensorsystems vorgesehen, dass die Signalaufnahmeeinheit zumindest teilweise oder vollständig biodegradierbar ist, also im tierischen/menschlichen Körper abbaubar ist. Hierzu können insbesondere die in der EP 2 433 562 B1 beschriebenen Maßnahmen verwendet werden. Auch im Hinblick mögliche Hydrogele, die im Rahmen der vorliegenden Erfindung verwendet werden können (siehe oben), wird auf die EP 2 433 562 B1 verwiesen.

Gemäß einem weiteren Aspekt der vorliegenden Erfindung ist ein Verfahren zum Bestimmen eines Merkmals, insbesondere zum Messen der Konzentration eines Analyten, insbesondere Glucose, in einem tierischen oder menschlichen Körper unter Verwendung eines medizinischen Sensorsystems nach einem der vorhergehenden Ansprüche vorgesehen, wobei ein magnetisches Wechselfeld zum Einwirken auf die Nanopartikel sowie Referenz-Nanopartikel ausgesendet wird und ein Antwortsignal in Form eines Relaxationssignals empfangen wird, das durch eine magnetische Wechselwirkung des magnetischen Wechselfelds mit den Nanopartikeln und den Referenznanopartikeln generiert wird, wobei mittels des empfangenen Relaxationssignals das betreffende Merkmal bestimmt bzw. nachgewiesen wird, wobei insbesondere die Konzentration des Analyten bestimmt wird.

Hierbei ist eine etwaige Implantation der Signalaufnahmeeinheit in einen tierischen oder menschlichen Körper ausdrücklich kein Bestandteil bzw. kein Verfahrensschritt des erfindungsgemäßen Verfahrens. Diesbezüglich wird davon ausgegangen, dass die Signalaufnahmeeinheit vor der Durchführung des erfindungsgemäßen Verfahrens auf bestimmungsgemäße Weise in einem tierischen oder menschlichen Körper angeordnet worden ist und somit dort bereits vorhanden ist. Das erfindungs gemäße Verfahren bezieht sich also insbesondere nur auf die Bestimmung bzw. den Nachweis des Merkmals, insbesondere auf die Konzentrationsbestimmung des fraglichen Analyten (z.B. Glucose oder die oben genannten weiteren Analyten).

Gemäß einer bevorzugten Ausführungsform des erfindungsgemäßen Verfahrens ist vorgesehen, dass zur Berechnung der Konzentration des Analyten zumindest der Imaginärteil der dynamischen Suszeptibilität der magnetischen Nanopartikel und der Referenz-Nanopartikel mit Hilfe des Antwortsignals für eine Vielzahl an Frequenzen des magnetischen Wechselfeldes berechnet wird, wobei zur Berechnung der Konzentration des Analyten ein Verhältnis zwischen der Amplitude eines den Referenz-Nanopartikeln zugeordneten ersten Peaks des Imaginärteils der dynamischen Suszeptibilität und der Amplitude eines den Nanopartikeln zugeordneten zweiten Peaks des Imaginärteils der dynamischen Suszeptibilität bestimmt wird.

Weitere Merkmale und Vorteile der Erfindung sollen nachfolgend anhand von Figurenbeschreibungen von Ausführungsformen der Erfindung beschrieben werden. Es zeigen:

Fig. 1    eine schematische Darstellung eines erfindungsgemäßen Sensorsystems;

Fig. 2    eine schematische Darstellung eines konkurrierenden Bindungssystems, das bei der vorliegenden Erfindung verwendet werden kann;

Fig. 3    den gemessenen Imaginärteil der dynamischen Suszeptibilität der Nanopartikel und Referenz-Nanopartikel über der Frequenz des anregenden magnetischen Wechselfeldes für verschiedene Konzentrationen des Analyten (hier Glucose); und

Fig. 4    das Amplitudenverhältnis $A_1/A_2$ der Peaks des Imaginärteils der dynamischen Suszeptibilität für verschiedene Analytkonzentrationen (Glucose).

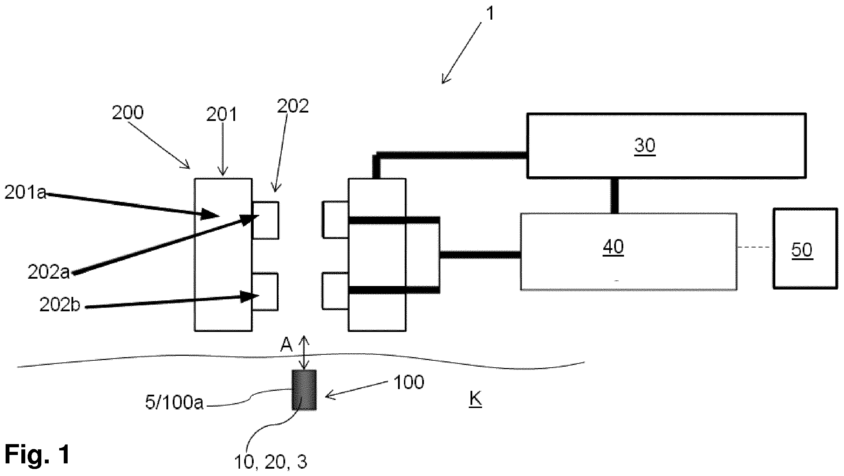

Figur 1 zeigt eine schematische Darstellung eines erfindungsgemäßen medizinischen Sensorsystems 1 zum Bestimmen eines Merkmals bzw. der Konzentration eines Analyten in einem tierischen oder menschlichen Körper. Im Folgenden wird die Erfindung exemplarisch anhand des Analyten 2 Glucose beschrieben. Wie oben bereits dargelegt, kann die Erfindung, insbesondere das Konzept eines Referenzsystems, jedoch auch im Zusammenhang mit anderen Merkmalen/Analyten verwendet werden.

Gemäß Figur 1 weist das Sensorsystem 1 eine Signalaufnahmeeinheit 100 mit einer Mehrzahl an magnetischen Nanopartikeln 10 auf, die jeweils dazu ausgebildet sind, durch Eingehen von reversiblen chemischen Bindungen mit einem weiteren Stoff 3, bei dem es sich hier um einen Rezeptor für Glucose, insbesondere Concanavalin-A (kurz: ConA), handelt, ihr magnetischen Relaxationsverhaltens zu ändern.

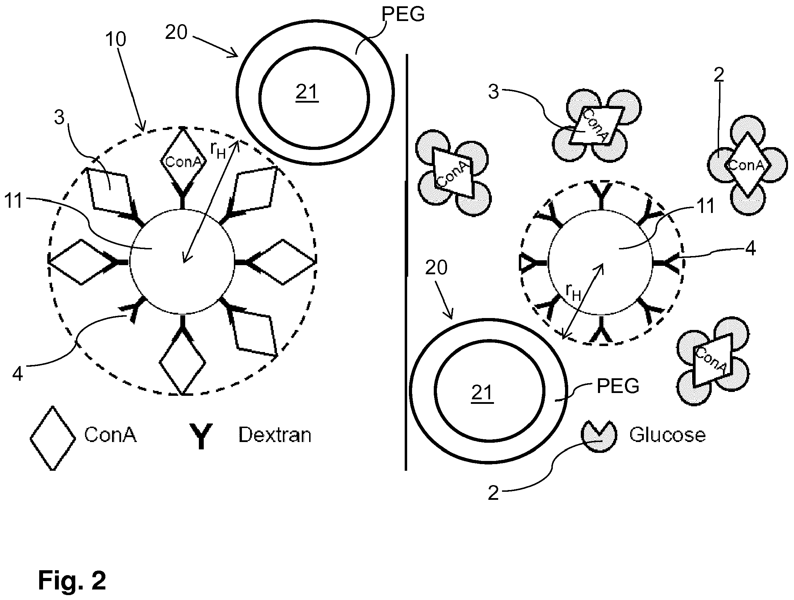

Hierzu kann gemäß Figur 2 ein Konkurrenzbindungssystem verwendet werden, wobei die magnetischen Kerne 11 der Nanopartikel 10 mit einem Analogon 4 des Analyten 2 Glucose beschichtet sind, bei dem es sich insbesondere um Dextran handelt. Vorzugsweise weist das Dextran ein Molekulargewicht zwischen 40.000 Da und 200.000 Da auf.

Ist die Konzentration des Analyten 2 im Bereich der Signalaufnahmeeinheit 100 niedrig, so binden sich vermehrt die Rezeptoren 3 ConA bzw. der weitere Stoff 3 an das Analogon 4 bzw. das jeweilige Dextran, wie es auf der linken Seite der Figur 2 gezeigt ist. Hierdurch wird der hydrodynamische Radius $r_H$ der Nanopartikel 10 entsprechend vergrößert, was sich auf die Brown-Relaxation auswirkt. Bei vergleichsweise hoher Analytkonzentration binden sich die Analyten/Glucose 2 an die Rezeptoren/ConA 3 und der hydrodynamische Radius $r_H$ der Nanopartikel 10 nimmt entsprechend ab (rechte Seite der Figur 2).

Zusätzlich weist das Sensorsystem 1 bzw. die Signalaufnahmeeinheit 100 weiterhin eine Mehrzahl an magnetischen Referenz-Nanopartikeln 20 auf, die die besagte chemische Bindung mit den Rezeptoren/ConA 3 nicht oder zumindest in einem hinreichend geringeren Ausmaß als die Nanopartikel eingehen. Bei den Referenz-Nanopartikeln handelt es sich z.B. um PEG, insbesondere um PEG300, d.h., um PEG mit einer Kettenlänge von 300 Monomeren.

Die Nanopartikel/Referenz-Nanopartikel 10, 20 sowie der Rezeptor 3 können gemäß Figur 1 in einem Hydrogel 5 (siehe auch oben) bereitgestellt werden, das dann zusammen mit den Komponenten 10, 20, 3 die Signalaufnahmeeinheit 100 bildet, die leicht in den tierischen bzw. menschlichen Körper K implantierbar bzw. injezierbar ist. Der Analyt 2 kann dann im Bereich des Hydrogels 5 in dieses hineindiffundieren, so dass die Reaktionen gemäß Figur 2 stattfinden können.

Gemäß einer weiteren alternativen Ausführungsform kann jedoch auch vorgesehen sein, dass die Signalaufnahmeeinheit 100 eine Hülle 100a aufweist, die von dem Analyten 2 bzw. Glucose durchdrungen werden kann, jedoch die Nanopartikel, Referenz-Nanopartikel sowie den Rezeptor 3 dauerhaft speichert bzw. umgibt. In diesem Falle diffundiert dann Glucose aus der Umgebung durch die Hülle 100a in die in den Körper K implantierte Signalaufnahmeeinheit 100, so dass die besagten Reaktionen gemäß Figur 2 in der Signalaufnahmeeinheit 100 stattfinden können.

Neben der in den tierischen oder menschlichen Körper K implantierbaren Signalaufnahmeeinheit 100 weist das Sensorsystem 1 weiterhin bevorzugt gemäß Figur 1 eine räumlich von der Signalaufnahmeeinheit 100 getrennt anordenbare, insbesondere ex vivo anordenbare, Signalverarbeitungseinheit 200 auf, die einen Sender 201 sowie einen Empfänger 202 aufweist, wobei der Sender 201 dazu ausgebildet ist, ein magnetisches Wechselfeld zum Einwirken auf die magnetischen Nanopartikel 10 und Referenz-Nanopartikel 20 der Signalaufnahmeeinheit 100 auszusenden, und wobei der Empfänger 202 dazu ausgebildet ist, ein Antwortsignal in Form eines Relaxationssignals der Signalaufnahmeeinheit 100 zu empfangen, das durch die magnetische Wechselwirkung des magnetischen Wechselfelds mit den magnetischen Nanopartikeln 10 und den magnetischen Referenz-Nanopartikeln 20 der Signalaufnahmeeinheit 100 generierbar ist. Der Sender 201 kann zum Erzeugen des magnetischen Wechselfeldes eine Spule 201a aufweisen, die geeignet mit einer Wechselspannungsquelle 30 verbunden sein kann. Der Empfänger 202, kann des Weiteren zwei Spulen 202a, 202b zum Empfangen der magnetischen Relaxationssignale aufweisen, wobei diese beiden Spulen 202a, 202b zur Auswertung der Relaxationssignale in bekannter Weise mit einem Lock-in-Verstärker 40 verbunden sein können, der das Signal der beiden Spulen 202a, 202b mit der Anregungsfrequenz 30 des Wechselspannungssignals multipliziert.

Der Abstand A zwischen der Signalaufnahmeeinheit 100 und dem Sender 201 bzw. der Spule 201a kann gemäß Figur 1 z.B. im Bereich von 1 bis 10 mm, vorzugsweise 5 mm, liegen. Das erzeugte magnetische Wechselfeld kann z.B. eine Amplitude im Bereich von 0 bis 600 $\mu$T Taufweisen.

Bevorzugt werden die Partikelgrößen bzw. Durchmesser der Nanopartikel 10, 20 so gewählt, dass die Neel Relaxationszeit bei Körpertemperatur von 37°C um drei Größenordnungen größer ist als die Brown Relaxationszeit. Dies ist insbesondere der Fall, wenn der besagte Durchmesser der Nanopartikel 10, 20 größer ist als ungefähr 11,2 nm.

Gemäß einem Beispiel der Erfindung werden als Nanopartikel 10 Nanopartikel mit einem Kern 11 aus ferromagnetischen Elementen wie Eisen, Kobalt, Nickel und/oder aus einer chemischen Verbindung dieser Elemente verwendet, der mit Dextran beschichtet ist, wobei der Durchmesser dieser Nanopartikel bei durchschnittlich 80nm liegt. Vorzugsweise weist das Dextran ein Molekulargewicht zwischen 40.000 Da und 200.000 Da auf.

Als Referenz-Nanopartikel 20 werden in diesem Beispiel Nanopartikel mit einem Kern 21 aus Eisenoxid verwendet, die mit PEG300 beschichtet sind, wobei der Durchmesser dieser beschichteten Referenz-Nanopartikel bei durchschnittlich 130nm liegt.

Die dynamische Suszeptibilität der Nanopartikel bzw. Referenz-Nanopartikel 10, 20 für den hier vorliegenden Fall der Brownschen Relaxation beträgt:

$$\chi = \frac{\chi_0}{1 + i\omega\tau_B}$$

wobei

$$\tau_B = \frac{4\pi r_H^3 \eta}{k_B T}$$

die Brown-Relaxationszeit ist. Hierbei rotiert das Teilchen 10, 20 mechanisch gegen die viskosen Kräfte des umgebenden Mediums (fluiddynamischer Prozess). Die Brown-Relaxationszeit ist proportional zur dritten Potenz des sogenannten hydrodynamischen Radius $r_H$ des Partikels, der in der Figur 2 eingetragen ist und maßgeblich durch die Bindung der Nanopartikel 10 zum Rezeptor 3 beeinflusst wird. Für den Fall einer geringen Glucosekonzentration binden sich entsprechend viele Rezeptormoleküle 3 ConA an das jeweilige Nanopartikel 10, so dass der hydrodynamische Radius $r_H$ steigt (linke Seite der Figur 2). Bei hoher Glucosekonzentration hingegen sinkt der hydrodynamische Radius $r_H$, da nun die Rezeptoren 3 ConA vermehrt durch die Glucose 2 gebunden sind.

Dies lässt sich durch Messung des magnetischen Relaxationsverhaltens der Nanopartikel 10 bzw. Referenz-Nanopartikel 20 auswerten.

Hierzu ist bevorzugt vorgesehen, dass der Empfänger 202, wie bereits angedeutet, zwei Spulen 202a, 202b aufweist, die gemäß Figur 1 insbesondere koaxial zueinander angeordnet sind, wobei in beiden Spulen 202a, 202b das magnetische Wechselfeld eine Spannung $V_{Spule1}$, $V_{Spule2}$ induziert, und wobei die Differenz $V_m = V_{Spule1} - V_{Spule2}$ dieser Spannungen gemessen wird.

In beiden Spulen 202a, 202b des Empfängers 202 wird dabei eine Spannung $V_{Anregung}$ induziert, die von dem magnetischen Wechselfeld abhängt sowie eine Spannung $V_{Partikel}$, die von der Magnetisierung der Nanopartikel bzw. Referenz-Nanopartikel 10, 20 stammt: $V_{Spule} = V_{Anregung} - V_{Partikel}$.

Da die Empfängerspulen 202a, 202b bevorzugt gemäß Figur 1 in der Senderspule 201a angeordnet sind, ist die induzierte Spannung $V_{Anregung}$ für beiden Spulen 202a, 202b gleich, so dass $V_m$ nur die Partikelbeiträge enthält. Sofern die Signalaufnahmeeinheit 100 nicht vorhanden ist, verschwindet $V_m$.

Mittels des Lock-In-Verstärkers 40 lässt sich nun bei vorhandener Probe bzw. Signalaufnahmeeinheit 100 in bekannter Weise ein Beitrag der Spannung $V_m$ bestimmen, der in Phase mit dem magnetischen Wechselfeld ist sowie ein Anteil, der um 90° dazu phasenverschoben ist (Imaginärteil von $V_m$).

Hierbei ergibt sich nun der Imaginärteil der oben eingeführten dynamischen Suszeptibilität zu

$$\chi = C \cdot \frac{V_m}{\omega}$$

wobei hier $V_m$ der Imaginärteil der mittels der Empfängerspulen 202a, 202b gemessenen Spannung $V_m$ ist, $\omega$ die Frequenz des magnetischen Wechselfeldes und C eine Konstante ist, die alle konstanten Faktoren beinhaltet.

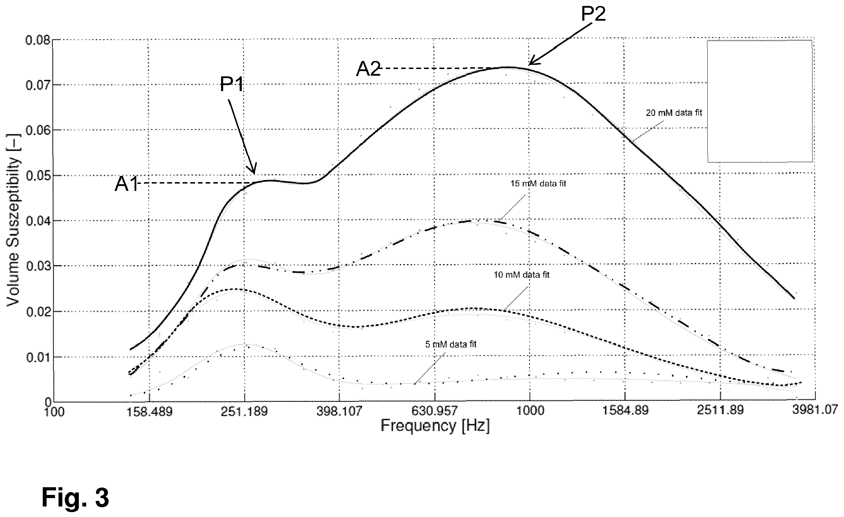

Es hat sich gezeigt, dass in dem in der Figur 2 gezeigten Bindungssystem bei niedriger Glucosekonzentration lange Teilchenketten bilden, die zu einer Sedimentation dieser Ketten führen. Dieses Sedimentationsphänomen im Bindungssystem der Figur 2 erweist sich als reversibel und schlägt sich deutlich in der gemessenen Amplitude des Imaginärteils der dynamischen Suszeptibiltät nieder, die in der Figur 3 für die oben spezifizierten Nanopartikel/Referenz-Nanopartikel 10, 20 für verschiedene Glucosekonzentrationen (5mM, 10mM, 15mM und 20mM) angeben ist.

Hierbei wurden im Einzelnen die folgenden Probenzusammensetzungen verwendet:

| Glucose [mM] | Nanopartikel 10 (80nm Dextran) [mg/ml] | Referenz-Nanopartikel 20 (130 nm PEG) [mg/ml] | Molares Verhältnis $c_p$ ConA/ Nanopartikel |
|---|---|---|---|
| 0-20 | 6 | 4,8 | 165 |

Die einzelnen verwendeten Probenvolumina setzten sich wie folgt zusammen (Komponenten 80nm Dextran (25mg/ml), 130nm PEG (10mg/ml), PBS (phosphatgepufferte Salzlösung) und Glucose (in PBS, 100mM):

| 80nm Dextran [$\mu$L] | 130nm PEG [$\mu$L] | PBS [$\mu$L] | ConA [$\mu$L] | Glucose [$\mu$L] | Gesamt vo lumen [$\mu$L] |
|---|---|---|---|---|---|
| 4,80 | 9,60 | 4,00 | 1,60 | - | 20,00 |
| 4,80 | 9,60 | 3,00 | 1,60 | 1,00 | 20,00 |
| 4,80 | 9,60 | 2,00 | 1,60 | 2,00 | 20,00 |

(fortgesetzt)

| 80nm Dextran [μL] | 130nm PEG [μL] | PBS [μL] | ConA [μL] | Glucose [μL] | Gesamt vo lumen [μL] |
|---|---|---|---|---|---|
| 4,80 | 9,60 | 1,00 | 1,60 | 3,00 | 20,00 |
| 4,80 | 9,60 | - | 1,60 | 4,00 | 20,00 |

Allerdings enthält das gemessene Signal bzw. die Amplitude des Imaginärteils der dynamischen Suszeptibilität gemäß Fig. 3 abgesehen von der Sedimentation auch Informationen über den Abstand der Messapparatur, über die Verdünnung der Partikel und die Anzahl der Partikel sowie die Viskosität.

Daher wird erfindungsgemäß das durch die Referenz-Nanopartikel 20 gebildete Referenzsystem verwendet und entsprechend das Verhältnis der ermittelten Amplituden $A_1$, $A_2$ betrachtet (siehe auch oben). Diese können ggf. durch eine Recheneinheit 50 automatisiert ausgewertet werden (vgl. Figur 1).

Fig. 3 zeigt z.B., dass bei einer Glucosekonzentration von 20mM zwei Peaks P1, P2 beobachtbar sind, wobei ein erster Peak P1 bei etwa 250 Hz und der zweite Peak P2 bei etwa 800 Hz (Wechselfeldfrequenz) auftritt.

Diese Peaks P1, P2 gehören zu den Nanopartikeln 10 bzw. Referenz-Nanopartikeln 20. Hierbei wird der Niedrigfrequenzpeak P1 durch die 130nm PEG Teilen 20 erzeugt, wohingegen der 800Hz-Peak P2 durch die 80nm Dextran Teilchen 10 erzeugt wird.

Die Relaxationssignale beider Partikelsorten 10, 20 überlappen, wobei die Amplitudenzunahme der Nanopartikel 10 bzw. der 80nm Dextran-Teilchen 10 dadurch erzeugt wird, dass diese von den ConA-Dextran-Ketten gelöst werden und dann zur Gesamtamplitude beitragen.

Da die Nanopartikel-Peaks P2 wesentlich starker steigen als die Referenz-Nanopartikel-Peaks P2 werden zur Glucosekonzentrationsbestimmung die Verhältnisse dieser Amplituden A1, A2 betrachtet:

$y=A_1/A_2=$(Amplitude gemessen bei 220Hz in Figur 3/Amplitude gemessen bei 894Hz in Figur 3).

Hieraus ergibt sich:

| Glucose [mM] | $A_1/A_2$ |
|---|---|
| 0 | 1,6048 |
| 5 | 1,1385 |
| 10 | 0,9910 |
| 15 | 0,9335 |
| 20 | 0,8920 |

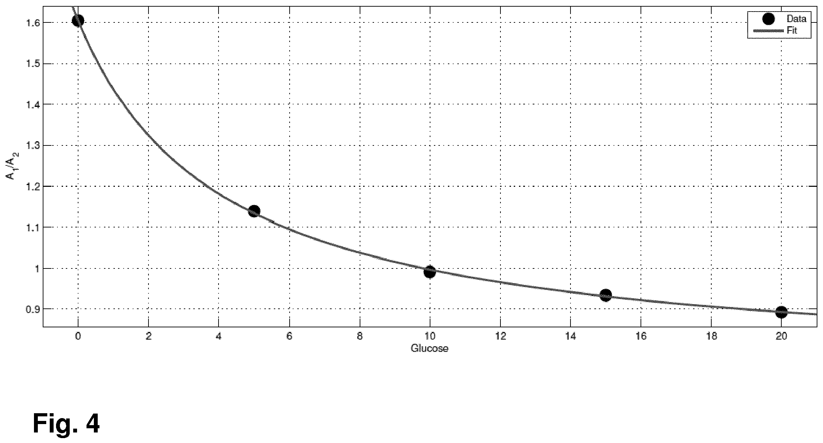

Die Verhältnisbestimmung $A_1/A_2$ erlaubt also grundsätzlich bei entsprechend kalibriertem Sensorsystem 1 eine Bestimmung der Glucose- bzw. Analysatkonzentration im Bereich der Signalaufnahmeeinheit 100, die aufgrund der Verwendung des erfindungsgemäßen Referenzsystems weniger störungsanfällig ist. Figur 4 zeigt einen Fit der Daten aus der vorstehenden Tabelle.

Der Signal-zu-Rausch-Abstand wurde durch wiederholte Messungen bestimmt. Die Standardabweichung von $A_1/A_2$ wurde zu 0,001377 bestimmt (22 Messungen).

In der Figur 4 ist das Verhältnis $A_1/A_2$ über der Glucosekonzentration dargestellt. Beide Partikelsorten 10, 20 reagieren mit ConA mit unterschiedlichen Reaktionsraten. In den Messungen (Fig. 3) überlagern sich die breitverteilten Peaks P1, P2.

Die Amplitude zu jedem Peak steigt zunächst linear an, so dass das Verhältnis $A_1/A_2$ an folgende Funktion gefitted wurde:

$$\frac{A_1}{A_2} = \frac{aG + b}{G + d}$$

wobei G die Glucosekonzentration *und a, b,* c Fitparameter sind. Das Ergebnis des Fits ist in Figur 4 gezeigt. Der gezeigt Fit weist einen $R^2$-Wert von 0,9998 auf und zeigt eine gute Übereinstimmung mit dem Modell. Die Nichtlinearität ergibt sich aus dem Umstand, dass beide Teilchensorten 10, 20 mit ConA wechselwirken und daher agglomerieren bzw. sedimentieren.

Um ein Sensorsystem 1 mit linearer Charakteristik zu erhalten, kann z. B. ein Referenz-Nanopartikel 20 verwendet werden, das nicht an ConA bindet. Weiterhin kann die Überlagerung der Brownschen Relaxation der beiden Partikelsorten 10, 20 von Einzelmessungen der beiden Partikel 10, 20 ermittelt und voneinander abgezogen werden:

$$y = \frac{A_2}{A_1 - a * A_2}$$

Hierbei enthält der Faktor *a* den Einfluss der Nanopartikel (80nm Dextran) auf $A_1$ sowie die Sedimentation der Referenz-Nanopartikel (130nm PEG). Für die vorliegenden Daten ergibt sich für *a*=0,75 ein lineares Verhalten.

## Patentansprüche

1. Medizinisches Sensorsystem (1) zum Bestimmen eines Merkmals in einem tierischen oder menschlichen Körper (K), mit
einer Mehrzahl an magnetischen Nanopartikeln (10), die jeweils dazu ausgebildet sind, durch Eingehen von reversiblen chemischen Bindungen mit einem weiteren Stoff (3; 4) des Sensorsystems (1) ihr magnetisches Relaxationsverhalten zu ändern,
**dadurch gekennzeichnet,**
**dass** das Sensorsystem (1) weiterhin eine Mehrzahl an magnetischen Referenz-Nanopartikeln (20) aufweist, die die besagten chemischen Bindungen nicht eingehen oder eine geringere Bindungsaffinität hinsichtlich dieser chemischen Bindungen aufweisen.

2. Sensorsystem nach Anspruch 1, **dadurch gekennzeichnet, dass** das Merkmal die Konzentration eines Analyten (2) ist, wobei die magnetischen Nanopartikel (10) jeweils dazu ausgebildet sind, in Gegenwart des Analyten (2) in Abhängigkeit von der Konzentration des Analyten (2) jene reversiblen chemischen Bindungen mit dem weiteren Stoff (3; 4) einzugehen, wobei diese Bindungen zu der Veränderung des magnetischen Relaxationsverhaltens des jeweiligen Nanopartikels (10) führen.

3. Sensorsystem nach Anspruch 2, **dadurch gekennzeichnet, dass** der weitere Stoff (3) ein Rezeptor für den Analyten (2) sowie für ein Analogon (4) des Analyten (2) bildet, wobei die magnetischen Nanopartikel (10) jeweils einen magnetischen Kern (11) aufweisen, wobei die Kerne (11) jeweils mit dem Analogon (4) beschichtet sind, so dass sich die magnetischen Nanopartikel (10) in Abhängigkeit von der Konzentration des Analyten (2) an den Rezeptor (3) des Analyten (2) und des Analogons (4) reversibel chemisch binden.

4. Sensorsystem nach Anspruch 2, **dadurch gekennzeichnet, dass** der weitere Stoff (4) ein Analogon (4) des Analyten (2) bildet, wobei die magnetischen Nanopartikel (10) jeweils einen magnetischen Kern (11) aufweisen, wobei die Kerne (11) jeweils mit einem Rezeptor (3) des Analyten (2) beschichtet sind, so dass sich die magnetischen Nanopartikel (10) in Abhängigkeit von der Konzentration des Analyten (2) an das Analogon (4) reversibel chemisch binden.

5. Sensorsystem nach Anspruch 3 oder 4, **dadurch gekennzeichnet, dass** das Analogon (4) ein Dextran ist und/oder dass der Rezeptor (3) Concanavalin-A ist.

6. Sensorsystem nach einem der Ansprüche 2 bis 5, **dadurch gekennzeichnet, dass** der Analyt (2) Glucose ist.

7. Sensorsystem nach einem der vorhergehenden Ansprüche, **dadurch gekennzeichnet, dass** die magnetischen Referenz-Nanopartikel (20) jeweils einen magnetischen Kern (21) aufweisen, der mit PEG, insbesondere mit PEG300, beschichtet ist.

8. Sensorsystem nach einem der vorhergehenden Ansprüche, **dadurch gekennzeichnet, dass** das Sensorsystem (1) weiterhin aufweist:

- eine in einen tierischen oder menschlichen Körper (K) implantierbare Signalaufnahmeeinheit (100), die die Nanopartikel (10), Referenz-Nanopartikel (20) und den besagten weiteren Stoff (3; 4) aufweist,
- eine räumlich von der Signalaufnahmeeinheit (100) getrennt anordenbare, insbesondere ex vivo anordenbare, Signalverarbeitungseinheit (200) mit einem Sender (201) und einem Empfänger (202), wobei der Sender (201) dazu ausgebildet ist, ein magnetisches Wechselfeld zum Einwirken auf die magnetischen Nanopartikel (10) und Referenz-Nanopartikel (20) der Signalaufnahmeeinheit (100) auszusenden, und wobei der Empfänger (202) dazu ausgebildet ist, ein Antwortsignal in Form eines Relaxationssignals der Signalaufnahmeeinheit (100) zu empfangen, das durch die magnetische Wechselwirkung des magnetischen Wechselfelds mit den magnetischen Nanopartikeln (10) und den magnetischen Referenz-Nanopartikeln (20) der Signalaufnahmeeinheit (100) generierbar ist.

9. Sensorsystem nach den Ansprüchen 2 und 8, **dadurch gekennzeichnet, dass** das Sensorsystem (1) eine mit der Signalverarbeitungseinheit (200) verbundene Recheneinheit (50) aufweist, die dazu ausgebildet ist, zur Berechnung der Konzentration des Analyten (2) zumindest den Imaginärteil der dynamischen Suszeptibilität ($X$) der magnetischen Nanopartikel (10) und der Referenz-Nanopartikel (20) mit Hilfe des Antwortsignals zu berechnen, wobei die Recheneinheit (50) dazu konfiguriert ist, zur Berechnung der Konzentration des Analyten (2) ein Verhältnis ($A_1/A_2$) zwischen der Amplitude ($A_1$) eines den Referenz-Nanopartikeln (20) zugeordneten ersten Peaks (P1) des Imaginärteils der dynamischen Suszeptibilität ($X$) und der Amplitude ($A_2$) eines den Nanopartikeln (10) zugeordneten zweiten Peaks (P2) des Imaginärteils der dynamischen Suszeptibilität ($\chi$) zu bestimmen.

10. Sensorsystem nach Anspruch 8 oder 9, **dadurch gekennzeichnet, dass**

- die Signalaufnahmeeinheit (100) zumindest ein Hydrogel (5) aufweist, in das der Analyt (2) hineindiffundieren kann, und das insbesondere von einem Copolymer gebildet ist, wobei die Nanopartikel (10), die Referenz-Nanopartikel (20) und der besagte weitere Stoff (3) in dem Hydrogel (5) angeordnet sind, oder dass
- die Signalaufnahmeeinheit (100) eine Hülle (100a) aufweist, durch die hindurch der Analyt (2) in die Signalaufnahmeeinheit (100) diffundieren kann, wobei die Nanopartikel (10), die Referenz-Nanopartikel (20) und der besagte weitere Stoff (3) von der Hülle (100a) umgeben sind.

11. Sensorsystem nach einem der Ansprüche 8 bis 10, **dadurch gekennzeichnet, dass** die Signalaufnahmeeinheit (100) zumindest teilweise oder vollständig biodegradierbar ist.

12. Verfahren zum Bestimmen eines Merkmals in einem tierischen oder menschlichen Körper (K), insbesondere zur Bestimmung der Konzentration eines Analyten (2) in einem tierischen oder menschlichen Körper, unter Verwendung eines Sensorsystems (1) nach einem der vorhergehenden Ansprüche, wobei ein magnetisches Wechselfeld zum Einwirken auf die Nanopartikel (10) sowie Referenz-Nanopartikel (20) ausgesendet wird und ein Antwortsignal in Form eines Relaxationssignals empfangen wird, das durch eine magnetische Wechselwirkung des magnetischen Wechselfelds mit den Nanopartikeln (10) und den Referenznanopartikeln (20) generiert wird, wobei mittels des empfangenen Relaxationssignals das Merkmal, insbesondere die Konzentration des Analyten (2), bestimmt wird.

13. Verfahren nach Anspruch 12, **dadurch gekennzeichnet, dass** zur Berechnung der Konzentration des Analyten (2) die dynamische Suszeptibilität ($\chi$) der magnetischen Nanopartikel (10) und der Referenz-Nanopartikel (20) mit Hilfe des Antwortsignals berechnet wird, wobei zur Berechnung der Konzentration des Analyten (2) ein Verhältnis ($A_1/A_2$) zwischen der Amplitude ($A_1$) eines den Referenz-Nanopartikeln (20) zugeordneten ersten Peaks (P1) des Imaginärteils der dynamischen Suszeptibilität ($\chi$) und der Amplitude ($A_2$) eines den Nanopartikeln (10) zugeordneten zweiten Peaks (P2) des Imaginärteils der dynamischen Suszeptibilität ($\chi$) bestimmt wird.

**Fig. 1**

Fig. 2

EP 3 251 595 A1

**Fig. 3**

Fig. 4

**Europäisches Patentamt**
**European Patent Office**
**Office européen des brevets**

# EUROPÄISCHER RECHERCHENBERICHT

**Nummer der Anmeldung**

EP 17 17 3392

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br><br>A | WO 2015/044027 A1 (SIEMENS AG [DE]) 2. April 2015 (2015-04-02)<br>* Abbildung 1 *<br>* Seite 13, Zeilen 15-16 *<br>* Seite 14, Zeilen 6-10 *<br>* Seite 14, Zeile 18 - Seite 15, Zeile 24 *<br>----- | 1-8,12<br><br>9-11,13 | INV.<br>A61B5/05<br>A61B5/145<br><br>ADD.<br>A61K49/18<br>B82Y5/00 |
| X<br><br>A | US 2014/295460 A1 (WEISSLEDER RALPH [US] ET AL) 2. Oktober 2014 (2014-10-02)<br>* Absätze [0118], [0127] - [0130] *<br>----- | 1-8,12<br><br>9-11,13 | |
| A | EP 2 433 562 A1 (BIOTRONIK SE & CO KG [DE]) 28. März 2012 (2012-03-28)<br>* Absätze [0039] - [0045] *<br>----- | 1-13 | |
| A | JOHN B WEAVER ET AL: "MAGNETIC SPECTROSCOPY OF NANOPARTICLE VIBRATION (MSNV) MEASUREMENT OF NANOPARTICLE BINDING",<br>PROCEEDINGS OF THE 2009 WORLD MOLECULAR IMAGING CONGRESS MONTREAL, CANADA,<br>Bd. 12, 3. November 2009 (2009-11-03),<br>Seiten S23-S23, XP055414371,<br>* abstract J040 *<br>----- | 1-13 | |
| | | | **RECHERCHIERTE SACHGEBIETE (IPC)**<br><br>A61B<br>A61K<br>G01N |

Der vorliegende Recherchenbericht wurde für alle Patentansprüche erstellt

| Recherchenort | Abschlußdatum der Recherche | Prüfer |
|---|---|---|
| Den Haag | 10. Oktober 2017 | Albrecht, Ronald |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 17 17 3392

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-10-2017

| Im Recherchenbericht angeführtes Patentdokument | Datum der Veröffentlichung | Mitglied(er) der Patentfamilie | Datum der Veröffentlichung |
|---|---|---|---|
| WO 2015044027 A1 | 02-04-2015 | DE 102013219114 A1<br>WO 2015044027 A1 | 09-04-2015<br>02-04-2015 |
| US 2014295460 A1 | 02-10-2014 | US 2014295460 A1<br>WO 2013078332 A1 | 02-10-2014<br>30-05-2013 |
| EP 2433562 A1 | 28-03-2012 | EP 2433562 A1<br>US 2012078068 A1 | 28-03-2012<br>29-03-2012 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2433562 B1